# EUROPEAN PATENT SPECIFICATION

(11) **EP 1 478 664 B1**
(45) Date of publication and mention of the grant of the patent: **03.11.2010**
(21) Application number: 03742543.6
(22) Date of filing: 19.02.2003
(51) Int. Cl.: C07K 14/705, C07K 14/47

(54) **TISSUE SPECIFIC EXPRESSION**
GEWEBSSPEZIFISCHE EXPRESSION
EXPRESSION HISTO-SPECIFIQUE

(30) Priority: 19.02.2002 DE 10206961
(43) Date of publication of application: 24.11.2004
(73) Proprietor: Oncoscience AG, 22880 Wedel (DE)
(72) Inventor: ALLGAYER, Heike, 81377 München (DE); LEUPOLD, Jörg, 81377 München (DE)
(74) Representative: Fleuchaus, Andrea
(86) International application number: PCT/EP2003/001671
(87) International publication number: WO 2003/070767

(56) References cited:
- ALLGAYER HEIKE ET AL: "Transcriptional induction of the urokinase receptor gene by a constitutively active Src. Requirement of an upstream motif (-152/-135) bound with Sp1." JOURNAL OF BIOLOGICAL CHEMISTRY, vol. 274, no. 26, 25 June 1999 (1999-06-25), pages 18428-18437, XP002243682 ISSN: 0021-9258 cited in the application
- DANG JINJUN ET AL: "A region between -141 and -61 bp containing a proximal AP-1 is essential for constitutive expression of urokinase-type plasminogen activator receptor." EUROPEAN JOURNAL OF BIOCHEMISTRY, vol. 264, no. 1, August 1999 (1999-08), pages 92-99, XP002243683 ISSN: 0014-2956
- LENGYEL ERNST ET AL: "Requirement of an upstream AP-1 motif for the constitutive and phorbol ester-inducible expression of the urokinase-type plasminogen activator receptor gene." JOURNAL OF BIOLOGICAL CHEMISTRY, vol. 271, no. 38, 1996, pages 23176-23184, XP002245129 ISSN: 0021-9258 cited in the application
- INASE NAOHIKO ET AL: "Use of gastrin-releasing peptide promoter for specific expression of thymidine kinase gene in small-cell lung carcinoma cells." INTERNATIONAL JOURNAL OF CANCER, vol. 85, no. 5, pages 716-719, XP002243685 ISSN: 0020-7136
- WILKE NORBERT ET AL: "Characterization of promoter elements mediating ethanol regulation of hsc70 gene transcription." JOURNAL OF PHARMACOLOGY AND EXPERIMENTAL THERAPEUTICS, vol. 292, no. 1, January 2000 (2000-01), pages 173-180, XP002243704 ISSN: 0022-3565

## Description

The present invention relates to a synthetic regulatory element for tissue specific gene expression. Furthermore, the invention relates to a system for a tumor cell specific expression of therapeutic genes as well as a pharmaceutical composition.

### Background of the Invention

Presently one of the main problems in clinical tumor therapy is that due to single tumor cells that remain at the side where a primary tumor was surgically removed, a new tumor starts to grow. The metastasis of tumor cells pre or after an operation leads to similar problems. In the last 20 years numeral experimental or clinical research reports demonstrated that malignant tumors invade tissues and develop metastases by the digestion of extracellular matrix structures and basal membrane (for review: Dvorak, 1986, Liotta, 1986). Digestion of extracellular matrix structures is mediated by various tumor associated protease systems (Schmitt et al., 1992), such as serine-, aspartic-, cysteine-, threonine- and metallo-proteases (Schmitt et al., 1992), which cause a degradation of matrix structures such as collagen or laminin by the initiation and promotion of proteolytic cascades as well as an interaction with different inhibitors. Thereby they allow tumor cells to invade the adjacent structures as well as the vascular system. The identity of the contributing enzymes does not differ from enzymes, which physiologically control wound healing, inflammation, embryogenesis or angiogenesis (Blasi, 1988 and 1993, Dano et al., 1985, Liotta, 1986, Liotta et al., 1991, Markus, 1988). It is the overexpression of these enzymes, which characterizes the invasive phenotype of malignant cells.

One of the best characterized tumor associated protease systems is the Urokinase (u-PA) system. The 55 kDa serine protease u-PA activates plasminogen to plasmin and degrades thereby directly and indirectly fibrin, fibronectin, proteoglycane, laminin and collagen type IV (the latter are components of the basal membrane (Andreasen et al., 1994, Duffy, 1992, Guenzler et al., 1982 a and 1982 b, Wun et al., 1982 a and 1982 b, Gronow and Bliem, 1993, Vassalli et al., 1984) thereby favourizing the invasion of cells in the adjacent structure.

The u-PA activity is modulated by various interaction of other proteases or inhibitory systems. On one hand these factors are activators or inhibitors of u-PA (cathepsin, α-1-antitrypsin, antithrombin III), substrates of u-PA (plasminogen, MMP-2/72kD-collagenase IV), parallel activators (t-PA) or antagonists of the plasminogen activating system by u-PA (α-2-macroglobulin) and cofactors which stabilize the specific u-PA receptors at the cell surface as well as its transcellular regulation (α-1-antichymotrypsin, α-2-macroglobulin (for review e. g. Schmitt et al., 1992, Allgayer et al., 1997)).

The specific activity of u-PA is many times amplified by the binding to the specific receptor (u-PAR). U-PAR, a 45 to 60 kDa glycosylated cell surface receptor concentrates the proteolytic effect of u-PA by its high flexibility in the membrane, which is due to a glycolipid anchor (Ellis et al., 1991, Behrendt et al., 1990, Estreicher et al., 1990, Ploug et al., 1991a, Moller et al., 1992). Receptor bound u-PA has a 40 times lower Kₘ for plasminogen than the free enzyme (Ellis et al., 1991).

The u-PA/u-PAR complex is internalized into a cell by the specific binding of an inhibitor, PAI-1, and the interaction with the α-2-macroglobulin receptor. Then the ligands will be degraded in lysosomes and the free u-PAR recirculates to the cell surface in a way that it is available for the binding with a new u-PA molecule (Olson et al., 1992, Cubellis et al., 1990, Conese et al., 1994, Nykiaer et al., 1992, 1994a, 1994b). This results in a flexible, dynamic and highly efficient proteolytic system.

Due to the close interaction between the u-PAR, its ligand u-PA and also the inhibitors PAI-1 or -2 the whole set up is likely to be called the "u-PA system" (Blasi, 1993).

The u-PAR comprises 3 similar protein domains (each with about 90 amino acids, Riittinen et al., 1996) and is physiologically expressed in small amounts in peripheral leucocytes, activated monocytes and T-lymphocytes, mature pre-leucocytes of the bone marrow and during wound healing at endothelial cells and ceratinocytes (Cubellis et al., 1990, Min et al., 1992, Vassalli et al., 1984, 1985, Plesner et al., 1994, Allgayer et al., 1997b, Langer et al., 1993, Romer et al., 1994a). Accordingly, the u-PAR plays an important role in processes like chemotaxis, migration, adhesion and in the signal transduction of the cytoskeletal building (May et al., 1998, Dewerchin et al., 1996, Pedersen et al., 1996, Resnati et al., 1996, Xue et al., 1997, Wie et al., 1994, 1996, Busso et al., 1994, Tang et al., 1998, Stahl and Müller, 1995, Bohuslav et al., 1995, Konakova et al., 1998, Yebra et al., 1996).

Furthermore, the u-PAR is overexpressed in various tumors of epithelial origin compared to non-tumor cells of the same origin. Primarily to mention are tumors of the gastrointestinal system (carcinoma of stomach or colon), carcinoma of the prostate gland or bronchial and ovarial carcinoma (Jankun et al., 1993, Sier et al., 1993, Pyke et al., 1991, Heiss et al., 1995b, Allgayer et al., 1998b, Cantero et al., 1997, Romer et al., 1994b, Morita et al. 1998, Jänicke et al., 1993, Nekarda et al., 1994).

For example in a colon carcinoma there is a clear correlation between the amount of endogenous u-PAR and the invasiveness of tumor cells (Hollas et al., 1991, Schlechte et al., 1989). Antibodies against the u-PAR were able to reduce the destruction of the extracellular matrix in HT29 cells about 80% (Reiter et al., 1993). Pyke et al. (1991a, 1991b) postulate that in colon carcinoma there is a paracrine interaction of the stroma cells surrounding a tumor, since they had observed an expression of u-PAR exclusively in tumor cells and a secretion of u-PA and PAI-1 in surrounding stroma cells.

According to some reports the quantity of u-PAR expression is therefore a criterion for the invasiveness or the malignant phenotype of cells (Wang et al., 1994, Bianchi et al., 1994, Hollas et al., 1991, Sliutz et al., 1996).

Additionally, various *in vitro* invasion models have shown that the transfection of a tumor cell line with u-PAR expression vectors results in an increased invasiveness in matrigel membranes or chorioallantoic membranes. In contrast thereto it was also shown that the inhibition of the u-PAR gene expression reduces the invasion of cells (Kariko, et al., 1993, Ossowski et al., 1988, Kook, et al, 1994, Quattrone, et al., 1995).

Furthermore, it was shown that an increased u-PAR expression stimulates tumor growth. It is believed that u-PAR combined with u-PA supports externally the invasion of the cell, while at the same time the combined u-PAR/u-PA launches a MAP-kinase signal inwards, which stimulates proliferation of the tumor cells (Aguirre-Ghiso, et al., 1999).

In summary the u-PA system seems an ideal starting point for a potential tumor therapy. For example the inhibition of the u-PA activity is seen as a promising therapeutic strategy not only regarding to an anti-invasive therapy (Quattrone, et al., 1995, Cavallaro, et al., 1993), but also with regard to a prevention of an establishment of minimal residual tumor cells to become metastases (Osborne and Rosen, 1994, Riethmüller, et al., 1992, Schlimok et al., 1990).

US 5,519,120 describes for example a therapeutic strategy to inhibit the u-PA system by monoclonal or polyclonal antibodies directed against the free u-PAR as well as complexes of u-PA and u-PAR. A disadvantage of this strategy is that the u-PAR, to which the u-PA is bound, but also the u-PAR, which is bound by a monoclonal antibody will be internalized on a regular basis and replaced by a newly synthesized u-PAR. This alone is already a reason why a therapy with antibodies can hardly provide continual success.

An alternative strategy is the inhibition of the u-PAR transcription and thereby the synthesis of u-PAR using antisense RNA (US 5,872,106; WO 96/03414).

Finally, in WO 98/46731, US 5,891,664 or EP 975743 peptides and polypeptides are disclosed, which inhibit the binding u-PA to u-PAR.

However, all this therapeutical strategies have not been finally successful. It is therefore an object of the present invention to provide a therapeutical strategy which allows a long-term inhibition of the u-PA system and thereby the treatment of recidivous tumors or metastasis.

### Detailed Description of the Invention

The invention suggests a molecular approach useful to activate a tissue specific expression of therapeutic genes. The tissue specific expression is mediated by synthetic regulatory elements deriving from natural occurring regulatory elements, which show an evidently higher activity in a specific tissue.

The invention provides a synthetic regulatory element namely promoter polymer (PP), which comprises one or more repetitions of a regulatory DNA sequence. The regulatory DNA comprises at least one transcription factor binding site.

The term "regulatory DNA sequence" is a nucleotide acid sequence not coding for any proteins but mediating by a specific sequence (transcription factor binding site) the binding with factors such as for example proteins, peptides, RNA molecules, other DNA molecules or more in general transcription factors. Such a binding causes a so-called trans-activation and results in the induction of gene expression. Trans-activation is particularly characterized by the influence of various factors on the transcription without a direct binding to the initiation site. Furthermore, there are factors, which inhibit gene expression via trans-activation (Lewin B., Genes: Chapter 29, Cell Press, Cambridge, 1990).

The term "promoter polymer" (PP) as used hereinafter describes a nucleotide acid sequence, which comprises at least one, preferable 3 to 7 or more repetitions of a promoter and/or regulatory sequences or parts thereof. Preferably the PP according to the invention is adjusted to the selection of transcription factors in the target tissue. This means that the regulatory DNA sequence according to the invention comprises one or more repetitions of transcription factor binding sites, which are involved in an increased gene expression in the target tissue.

It is common knowledge of the skilled person how to identify upregulated genes in a specific target tissue and further to identify and determine the tissue specific transcription factors by applying a mobility analysis using consensus sequences or natural sequences of transcription factor binding sites. Due to this the invention is broadened to the application on and/or therapy of diseases, which are characterized by an increased trans-activated gene expression. Particularly, the invention is useful to treat various tumors or inflammatory processes.

The synthetic PP according to the invention induces when introduced in a target tissue of interest an increased gene expression via the repetition of transcription factor binding sites.

This gene expression is highly specific for the tissue of interest while in the surrounding tissue no essential gene expression is introduced via the PP of the invention. In case that the PP according to the invention controls the expression of a therapeutic gene that gene will be essentially expressed only in the tissue of interest but not in the surrounding tissue.

A further advantage of the invention is that the PP according to the invention binds freely available transcription factors, particularly such factors, which are involved in a tumor cell specific up regulated gene expression. This results in a competition between the PP according to the invention and the natural promoter thereby down regulating the normal tissue specific gene expression.

According to further embodiments the present invention uses, particularly - but not exclusively - binding sites of the transcription factor AP1 as well as AP1 family members, c-Fos, c-Jun, JunD or Fra-1, further AP-2, particularly AP-2α or the clearly distinguishable AP-2-similar (Allgayer et al., 1999a), Sp1, Sp3 or Sp4 (for general information about transcription factors and their functionality see: Lewin, Genes VII, Cell Press, Cambridge, Mass., 2001).

In a further embodiment the PP according to the invention comprises one or more transcription elements deriving from the genes encoding the human urokinase receptor (u-PAR) promoter.

The u-PAR gene comprises 7 exons and is located on chromosome 19q13 (Borglum et al., 1992, Vagnarelli et al., 1992, Casey et al., 1994). Transcription of the gene results in a 1.4 kilo base mRNA or an alternatively spliced variant, which has no membrane localization signal and, thereby, is secreted (Roland et al., 1990, Pyke et al., 1993). Compared to other housekeeping genes the u-PAR gene does not comprise a TATA-box or CAAT-motives (Soravia et al., 1995). Instead there are multiple Sp1 consensus elements proximal to the main initiation point of the transcription (Soravia et al., 1995). These multiple Sp1 elements are responsible for the basal promoter activity. It was shown for the colon carcinoma model that by using DNAse-1-foot-printing besides the proximal regulatory region with Sp1 elements (region 3, -99/-70) two further promoter regions were protected by hybridization of the nuclear extract of RKO cell line (a colon carcinoma cell line with a high u-PAR expression), which indicates the binding of further transcription factors:
There is one distal region (-190/-171, region 1) with an AP-1 consensus motive (activated by Jun/Fos-proto-oncogenes) which plays a role in the constitutive and PMA inducible u-PAR promoter activity. A binding of this region further mediates an u-PAR induction by the k-ras oncogene, which is mutated in about 70% of all colon carcinoma (Allgayer et al., 1999c).

The second region (-148/-124, region 2) comprises a potential AP-2 and Sp1 element. Again for the colon carcinoma model it was shown that this region can be bound by a potentially new isoform (AP-2-similar) of the transcription factor AP- 2α as well as from Sp1 and Sp3. The binding of the AP-2-similar protein mediates a constitutive as well as a phorbol ester (PMA) inducible u-PAR gene expression. The binding of the Sp1 transcription factor group mainly mediates the PMA inducible gene expression. Sp1 further mediates the induction of u-PAR by the src-oncogene, which is over-activated in almost 80% of all colon or stomach carcinoma.

In one embodiment the synthetic PP according to the invention comprises one or more repetitions of the sequence regions -152/-128, -152/-130, -152/-135, -154/- 128, -154/-130 or -154/-135 (referring to the transcription initiation site) of the regulatory region of the u-PAR promoter. In further embodiments each PP according to the invention comprises one or more repetitions of the sequence regions -148/-124 and/or - 190/-171 (referring to the transcription initiation site) of the regulatory elements of the u-PAR promoter. In still another embodiment the synthetic PP according to the invention comprises two or more repetitions selected from the following group of sequence regions -190/-171, - 148/-124, -152/-128, -152/-130, -152/-135, -154/-128, - 154/-130 and -154/-135. According to a further embodiment of the invention DNA spacer sequences are introduced between the repetitions of regulatory DNA sequences allowing to increase the grade of polymerization. Such spacers can have a size of 2 to 20 nucleotides and prevent interactions of the transcription binding sites with unspecific factors.

To further distinguish promoter polymers, which are based on alternative promoter regions, the relevant transcription factor binding sites can be identified by screening existing databases and, subsequently, verifying in mobility shift analysis with nuclear extracts of 2 representative cell lines that the desired transcription factor will actually bind the polymer. Additionally, the polymer will be tested in cell lines, in which the original gene of the used promoter is either overexpressed or down-regulated.

According to a further embodiment the present invention provides a vector containing a therapeutic gene and/or a marker or reporter gene under the regulatory control of the PP according to the invention.

In general the term "vector" describes a circular DNA vehicle for example a plasmid, cosmid or artificial chromosome, which besides the desired DNA comprises regulatory sequences, a selection or marker gene and an origin of replication.

Therapeutic genes according to the invention are genes, which are capable of destroying a tumor cell when expressed. Useful are particularly genes, which induce apoptose of a tumor cell for example the p53 gene. Additionally useful are genes, which induce cell death only after the addition of a certain activator (Herpes simplex thymidine kinase gene, cytochrome p450 gene). Therapeutic genes according to the present invention or also marker and reporter genes are genes, which can introduce a color marker into a tumor cell (LacZ - beta-galactosidase gene or EGFP - Green Fluorescent Protein Gene) or which mark a tumor cell for defensive cells of the immune system by the expression of cytokines, specific antigenes or epitopes for therapeutic antibodies.

The vector according to the invention, comprising a therapeutic gene under the regulatory control of the PP according to the invention, is suitable to be administered as direct therapeutical treatment into a patient, for example as DNA vaccine. Furthermore, the vector according to the invention can be applied as cream for topical application.

In a further embodiment of the present invention said vector can be used for the generation of recombinant virus. Vectors, which are useful for the generation of recombinant virus comprise additionally virus-specific sequences, which mediate a homologous recombination between the vector and the selected virus. For the recombination the vector and the selected therapeutic useful virus will be jointly introduced in a suitable cell expression system and after an appropriate incubation time recombinant virus can be isolated and purified with suitable selection methods.

Preferred viruses according to the invention are viruses, which are capable to integrate the therapeutic gene under the transcriptional control of the PP according to the invention into the genome of a tumor cell. The stabile insertion into the genome of the tumor cell guarantees a long-lasting expression. Preferably - but not exclusively, adeno-associated viruses or retroviruses will be used according to the invention.

Depending on the tissue, which one wants to infect, useful viruses according to the invention are vaccinia viruses, adenoviruses, which particularly infect eyes, respiratory tissue and colon tissue or lentiviruses like MMTV with its increased affinity to mammary tissue. By the selection of the different viruses the various tissues such as carcinoma of the lung or mamma can be directly infected and individually treated.

In a further embodiment the present invention provides a pharmaceutical composition which comprises the vector or recombinant virus according to the invention and one or more pharmaceutical acceptable carriers, additives, antibiotics, conservatives, adjuvants, diluents and/or stabilizers. Such additives can be: water, saline, glycerol, ethanol, pH buffering substances or the like. Suitable carriers are normally big and slowly metabolized molecules such as for example proteins, polysaccharides, polymeric amino acids, lipid aggregates or the like.

In a further embodiment the present invention provides a vaccine comprising a recombinant virus with synthetic PP in a therapeutical effective concentration as well as optional additives such as mannitol, dextran, sugar, glycin, lactose, polyvinyl pyrrolidone, antioxidants or recombinant proteins (Albumin) which are suitable for an *in vivo* application. Typical routes of application are known to the skilled person and are for example a topical, intraperitoneal or systemic and also intratumorale application.

### Short Description of the Figures

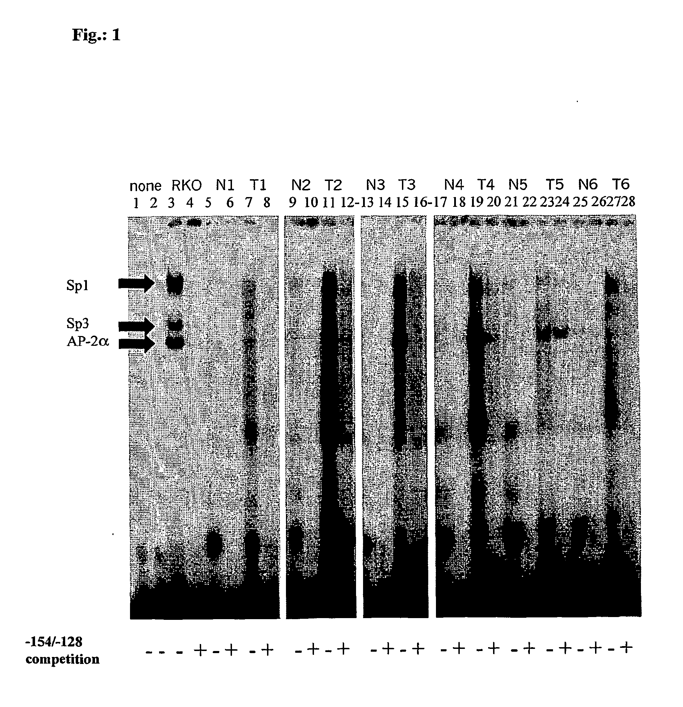

- **Figure 1**: shows a motility shift experiment approving the binding of the transcription factors AP-1- similar protein, Sp1 and Sp3 to the region -152/-130 of the u-PAR. Lane 1 and 2 are negative controls (none). Lane 3 shows the binding of the transcription factors AP-2- similar protein, Sp1 and Sp3 (arrow), which are present in a defined amount of the nuclear extract of the standardized colon carcinoma cell line (RKO). The binding of the transcription factors was highly specific in cells of the normal tissue (N) and the tumor tissue (T) of a carcinoma patient because it was shown that a 200-time surplus of an unmarked consensus oligonucleotide could compete the transcription factors. Lanes 4, 6, 8, 10, 12, 14, 16, 18, 20, 22, 24, 26 and 28 are marked with a "+" to draw the attention to the vanished band of the transcription factor.

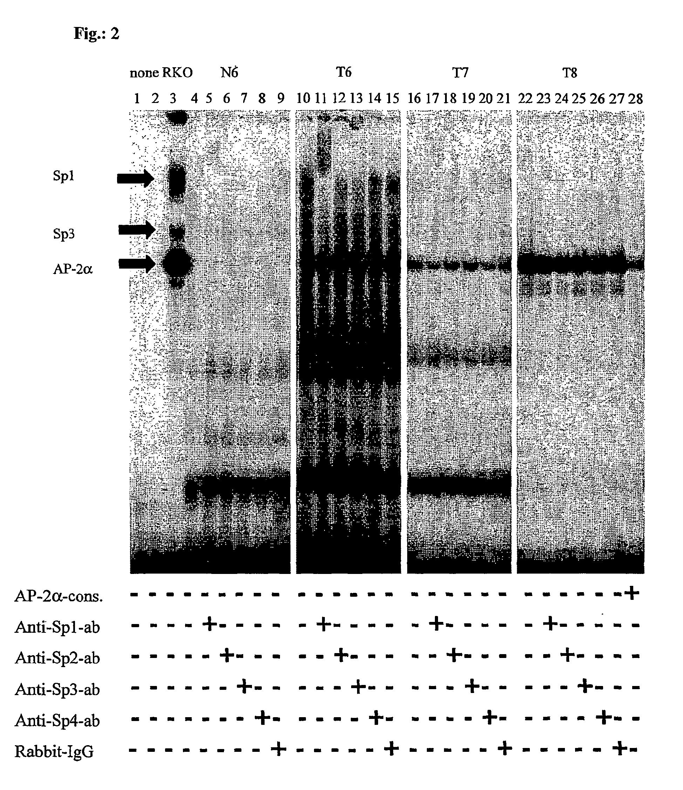

- **Figure 2**: shows a supershift experiment, which proves the binding of the transcription factors AP-2- similar protein, Sp1 and Sp3 to the region -152/-130. Lane 1 and 2 are negative controls (none). Lane 3 shows the binding of the transcription factors AP-2-similar protein, Sp1 and Sp3 are shown (arrow), which are present in a defined amount in the nuclear extract of the standardized colon carcinoma cell line (RKO). The specificity of the AP-2 band was confirmed for nuclear extracts from several tumor preparation (T) and one normal tissue preparation (N) by using a 200 times surplus of an unmarked AP-2 consensus oligonucleotide (lane 28). The specificity of the Sp3 band was confirmed by using an antibody directed against the DNA binding domain of the Sp3 (lane 19). The specificity of the Sp1 band was confirmed by using an antibody directed against Sp1 (lane 11 and 17).

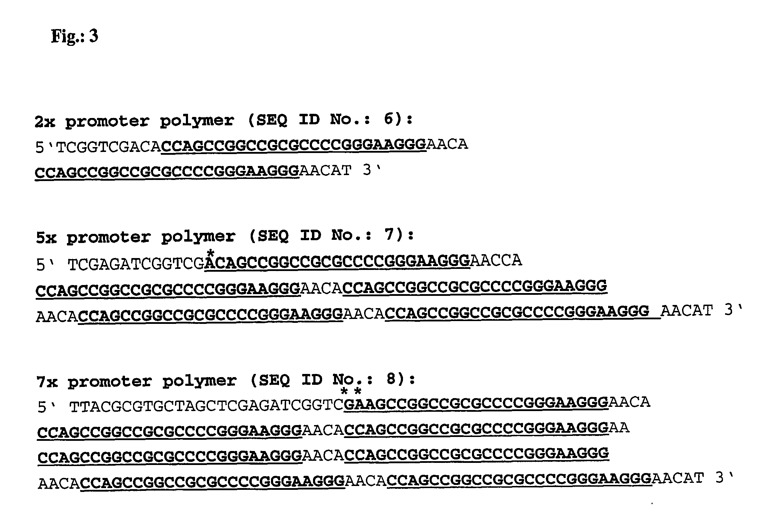

- **Figure 3**: shows the sequence of various synthetic promoter polymers comprising the region -154/-128 of the human u-PAR promoter, which contains the AP-2 and Sp1 transcription factor binding site. The sequences are marked with asterix at the location were a base pair exchange occurred during the PCR amplification.

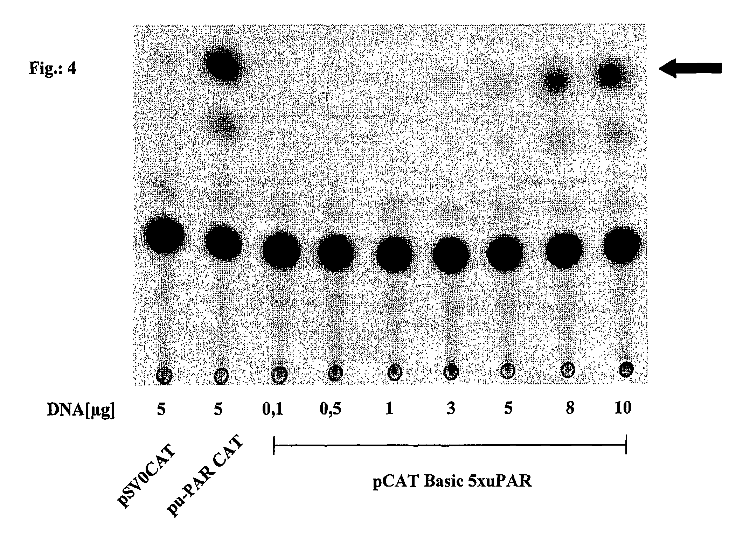

- **Figure 4**: shows the transcription activity of the promoter region -154/-128 of the human u-PAR promoter in RKO cells. pSVOCAT stands for a control plasmid, which comprises a chloramphenicol gene without transcriptional control element. Pu-PAR CAT stands for a control plasmid, which comprises a chloramphenicol gene under the transcriptional control of the natural occurring human u-PAR promoter. pCAT basic 5x u-PAR stands for a vector plasmid, which comprises the chloramphenicol gene under the transcriptional control of a promoter polymer with a 5x repetition of the region -154/-128 of the u-PAR promoter. Lane 2 shows the CAT expression of the natural promoter in RKO cells. Lane 3 to 9 show the CAT expression of the vector comprising the promoter polymer in a concentration depending manner.

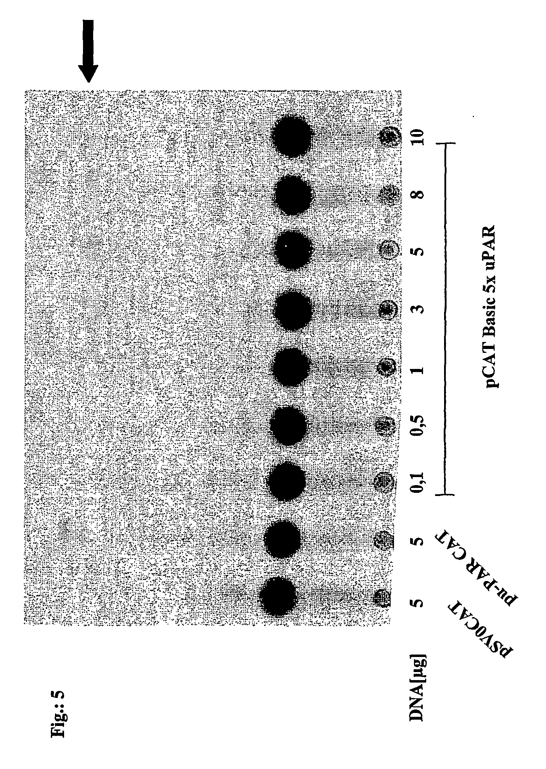

- **Figure 5**: shows the transcription activity of the promoter region -154/-128 of the human u-PAR promoter in GEO cells. pSVOCAT stands for a control plasmid, which comprises a chloramphenicol gene without transcriptional control element. Pu-PAR CAT stands for control plasmid, which comprises a chloramphenicol gene under the transcriptional control of the natural occurring human u-PAR promoter. pCAT basic 5x u-PAR stands for a vector plasmid, which comprises the chloramphenicol gene under the transcriptional control of a promoter polymer with a 5x repetition of the region -154/-128 of the u-PAR promoter. It can be clearly shown that none of these constructs can induce a CAT activity in the used cell lines.

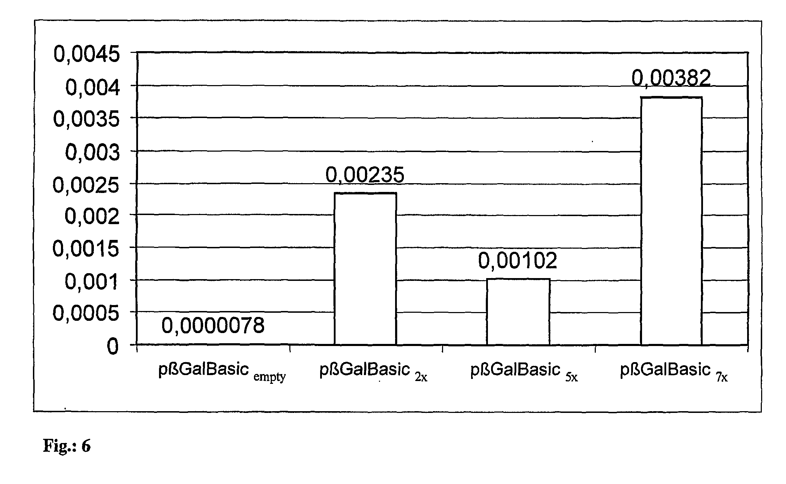

- **Figure 6**: shows in a beta-galactosidase assay the transcription activity of the various promoter polymers of the promoter region -154/-128 of the human u-PAR promoter in RKO cells. PβGalBasic- empty stands for a vector without any promoter. By the additional indication of 2x, 5x, 7x the grade of polymerization of the PP in various vectors is indicated.

### Examples

### Example 1: Analysis of the u-PAR promoter region

Urokinase receptor gene (u-PAR) essentially contributes to the invasion and metastasis of various tumors. Compared with normal tissue the expression of u-PAR is regulated above average in tumor cells.

Studying the u-PAR promoter region lead to a detailed characterization of several independent regions, to which various factors bind and which are essentially transcription involved in the regulatory control of the u-PAR expression. All these studies took place in tumor cell lines *in vitro*, however, the regulatory control of this promoter region was also confirmed *in vivo.* Therefore, the gastrointestinal tumor tissue of 145 patients was examined in mobility assays to examine the regulation of the u-PAR expression by the promoter region, which binds AP-2/Sp1.

The mobility analysis or electrophoretic mobility shift assay (EMSA) as well as the extraction of nuclear extracts was carried out as described earlier by Lengyel et al (1996) and Dignam et al. (1983). For the isolation of nuclear extracts from patient tissue, a piece of tumor tissue and a piece of normal tissue of a patient as well as control tissue of a non tumor patient was isolated and frozen in liquid nitrogen. The frozen tissue was mechanically grinded, suspended in 1M PBS buffer and for the extraction of nuclear extracts treated as follows:

For the isolation of the nuclear extract the tissue pieces and also cells of the control cell line RKO were centrifuged, incubated an ice for 10 minutes in a buffer of 10 mM HEPES pH 7.9, 10mM KCI, 1.5 mM MgCl2, 0.5 mM DTT, 0.2 mM PMSF and 2 mg/ml aprotinin (Sigma, St. Louis, MO, USA), shacked and again centrifuged. The pellet comprising the nuclei were resuspended in a buffer of 20 mM HEPES, pH 7.9, 0.45 M NaCl, 1.5 mM MgCl2, 25% Glycerol, 0.2 mM EDTA, 0.5 mM DTT, 0.5 mM PMSF and 2 mg/ml aprotinin and was shacked for 30 minutes at 4° C. Storage of the nuclear extracts took place at -80° C.

Per reaction 25 µg of the nuclear extract and 20.000 cpm of a γ ³²P-ATP endmarked oligonucleotide (T4-phagen-oligonucleotid-kinase, Boehringer Mannheim) corresponding to the region -152/-135 of the u-PAR promoter (5'-CCA GCC GGC CGC GCC CCG GGA AGG GA-3'; SEQ ID No.: 9) were incubated for 15 minutes in a buffer of 25 mM HEPES, pH 7.9, 0.5 mM EDTA, 0.5 mM DTT, 0.5 M NaCl, with or without 200 times surplus of non-marked oligonucleotides as competitor. Each reaction additionally comprised 0.5 µg Poly(dIdC) for blocking of unspecific reactions. The reactions were separated in a 5% polyacrylamid gel (5% glycerol) at room temperature and exposed at a Kodak MR film for 24 hours at -80° C.

As result the mobility analysis showed that a strong binding of the transcription factors AP-2α and AP-2 similar. Sp1 and Sp3 was essentially involved in the regulation of the u-PAR expression in above 60% of the tumors (Fig. 1). The supershift experiment confirmed that actually the same transcription factors in the patient tissue as well as in the control cell line are responsible for the shift (Fig. 2).

For this supershift analysis after 10 minutes of incubation 1 µg of a specific antibody against a transcription factor (for example anti-Sp1, -Sp2, -Sp3, - Sp4 and rabbit IgG control, Santa Cruz Biotechnology catalogue no. sc-59, sc-643, sc-644, sc-645, sc-2338) were added to the nuclear extract and the oligonucleotide and further incubated for 60 minutes on ice. Electrophoresis runs at 4° C to stabilize the emerged complexes. The addition of AP-2 consensus oligonucleotides with the sequence 5'-GAT CGA ACT GAC CGC CCG CGG CCC GT-3', SEQ ID No.: 10 (catalogue no. sc-2513, Santa Cruz Biotechnology) displaced the binding of the AP-2 similar factors. For each mobility shift assay was used a negative control without nuclear extract and a second without nuclear extract and poly(dIdC).

As result of the mobility shift it could be shown that in 68 of 117 (58,1%) of colorectal cancer patients a strong transcription factor binding of Sp1 was detected compared to the low and non-existing binding in normal tissue. The same is true for the binding of Sp1 in 15 of 28 (53,6%) of stomach cancer patients. The binding of the AP-2α-similar factor it was possible to detect in 69 of 117 (59,0%) patients having colon cancer and further in 18 of 28 (64,3%) patients having stomach cancer. A binding of both tested factors, namely AP-2α and Sp1, was shown that in 61 of 117 (52,1%) patients having colon cancer and in 11 of 28 (39,3%) patients having stomach cancer. There was a significant correlation (p<0,0001, p=0,0003, linear regression) of the binding of AP-2α-similar proteins and Sp1 factors with the amount of u-PAR protein in the tumor tissue (ELISA). However, there was no such correlation in normal tissue. These results allow the conclusion that the trans-activation of the u-PAR expression using this promoter region or the PP according to the invention *in vivo* would only be relevant in tumor tissue.

These results further suggest that for about 60% of all patients having a gastrointestinal tumor, wherein a trans-activation of the u-PAR gene region in the tumors compared to the normal tissue was implicated by the region -152/-135, a molecular targeting of this region using the PP according to the invention would be possible.

Further it is possible to use for a molecular cell targeting a combination of this strategy together with an additional inhibiting of pathways, which are also activated by this promoter region (like Src-inhibition, which is mediated by using the Sp1 binding site in this promoter region).

Additionally, it is also possible to use therapeutic strategies with further polymerized promoter elements such as a polymer for example of the AP-1 region (-190/- 171), particularly, when in an individual case both these promoter elements were identified as being involved in the tumor specific regulation.

### Example 2: Construction of a vector comprising a promoter polymer

To test, which grade of polymerization would result in an optimal gene expression several vectors were constructed, which comprise a 1 to 10-time repetition of the promoter region and also a suitable reporter gene for an *in vitro* testing. Preferably the beta-galactosidase gene (lacZ) or the chloramphenicol-acetyl-transferase gene (CAT) was used for *in vitro* tests.

For the construction of a CAT reporter comprising a promoter polymer of region 2 an oligonucleotide corresponding to the base pairs -154/-128 of the u-PAR promoter was synthesized. To the 5' end of the sense-strang of this oligonucleotide (position -154) the nucleotides C and A were added and at the 5' end of the non-coding strang (position -128) the nucleotides G and T were added. The resulting synthetic oligonucleotide (oligo) with the sequence 
5' - CAC CAG CCG GCC GCG CCC CGG GAA GGG AA -3'
3- G GTC GGC CGG CGC GGG GCC CTT CCC TTG T -5'
(5'->3': SEQ ID No.: 2) (3'->5' SEQ ID No.: 3)
 was purified by using PAGE and resuspended in Aq dest with the final concentration of 1 µg/pl.

The single strangs were annealed at 90° C for 2 minutes (4 µl coding, 4 µl non coding strang and 64 µl Aq dest) and immediately after the annealing 8 µl 1N NaCl was added to stabilize the resulting double strang. The resulting oligo concentration was 100 ng/µl.

For the ligation of the oligos to form polymers of various lengths it was used: 15 µl ligase (Biolabs 202S, 400.000 U/ml), 20 µl oligo (2 µg), 7 µl ligase buffer (Biolabs T4 DNA ligase buffer with 10 mM ATP) and 28 µl Aq dest. The ligation was performed for 14 hours at 16° C. The resulting ligated products were purified using the phenol/chloroform method with 3 M NaCOOH/100% ethanol for 30 minutes at -70° C, washed with 70% ethanol, vacuum dried and resuspended in 10 µl TE buffer.

The CAT vector (Promega pCAT vector) was digested and linearized with SalI, purified as described above and eventually resuspended in 20 µl Aq dest. The oligo nucleotides as well as the linearized pCAT vector were blunded by using Klenow enzyme and dNTPs, was again purified, precipitated, dried and resuspended as described above (the oligos were resuspended in 20 µl TE to reach a final concentration of 50 ng/µl, the vector was resuspended in 40 µl Aq dest). Further the pCATbasic vector was dephosphorylated with alkaline phosphatase to prevent autoligation and again phenol/chloroform purified, precipitated as described above and resuspended in Aq dest to reach a final concentration of about 100 µg/µl. The polymers were diluted in TE to reach a final concentration of 25 µg/µl.

Various reactions with different ratio of polymer/vector were started to clone the polymers into pCAT:

| Reaction: # | 0 | 1 | 2 | 3 | 4 | 5 | 6 | 7 |
|---|---|---|---|---|---|---|---|---|
| PCAT (µl) | 2 | 1 | 1 | 1 | 1 | 1 | 1 | 1 |
| Polymer (µl) | 0 | 1 | 2 | 3 | 4 | 5 | 6 | 0.5 |
| 10 times ligase buffer (µl, Biolab) | 1 | 1 | 1 | 1 | 1 | 1 | 1 | 1 |
| Aq dest | 5 | 5 | 4 | 3 | 2 | 1 | 0 | 5.5 |
| T4-ligase (µl, Biolab) | 2 | 2 | 2 | 2 | 2 | 2 | 2 | 2 |

The ligation took place for 24 hours at 4° C. To stop the reaction 20 µl TE buffer were added to each reaction.

The ligation products were transformed into *E. coli,* multiplied and sequenced. As a result there were various vectors with different grades of polymerization in sense orientation.

For the functional assays 3 constructs with a 2-times, 5-times and 7-times repetition of the above described oligonucleotide sequence were selected. Furthermore, 2 promoter polymers with a 5-times and 7-times repetition were tested, which had incorporated base pair exchange during PCR amplification (Fig. 3). However, no functional difference could be shown.

In a mobility shift analysis in which these polymers were marked radioactively, it was shown that the known transcription factors are able to bind the polymers.

It is not possible in a mobility shift analysis to directly and quantitatively compare the transcription factor binding of the various constructs between each other and in comparison to the simple promoter element, since due to the different lengths of the different constructs the resulting DNA/protein complexes show a different behavior due to a variation in their relative weight in the gel. However, it was possible to show in mobility shift analysis that the AP-2 similar and Sp1 transcription factor are able to bind these polymers.

### Example 3: Mobility shift with various polymers

The polymers were cut free from pCATBasic with PstI/XbaI, sticky ends were filled with dNTPs using T4-DNA-polymerase (New England Biolabs) and radioactively marked with γ-³²P-ATP (ICN) using T4-polynucleotid kinase. For one mobility shift reaction 20.000 cpm radioactively marked polymer were used. As described above the mobility shift were performed by using 15 µg nuclear extract of RKO cell line and 0,2 µg poly(dIdC) in each reaction. The reactions were separated in a 4% polyacrylamid gel (5% glycerol). AP-2 consensus as well as Sp1 consensus in a 100 times surplus eliminated the AP-2 as well as the Sp1 band.

Functionality of the various polymers was proved in a reporter assay.

### Example 4: CAT (chloramphenicol-acetyl-transferase) Assay

As control a plasmid was used, which carries the natural occurring promoter region of the u-PAR gene. This plasmid was generated by cloning a 449 base pair long sequence (Wang et al., 1995), which comprises the position -398 to + 51 relatively to the main transcription initiation site of the u-PAR gene, into a CAT as well as a luciferase reporter plasmid (pCAT basic vector (Promega, Madison, WI, USA) was cut at the XbaI restriction site; PGL3 vector (Promega) was cut at SmaI site.

The expression of these plasmid vectors, which comprise a promoter polymer with a 5-time repetition of region 2 (base pair -154/-128), was tested in 2 different cell lines. On one hand side a slightly differentiated colon carcinoma cell line, RKO (ATCC: CRL-2577) with a high endogene u-PAR expression was used. The second cell line was also a colon carcinoma line, GEO, which naturally shows non or only a very low u-PAR expression (Brattain et al., 1984). Alternatively, was used a transformed human renal cell line 293 (ATCC: CRL-1573), which has a low u-PAR expression.

The various cells were grown to 60% confluency and transfected following a method of Nead and McCance (1995) using poly-L-ornithin (Boehringer). The transfection took place for 6 hours using poly-ornithin/DNA in a ratio of 0.47 with 0.1 to 10 µg of the relevant plasmid DNA in serum-free medium. In case of the CAT assay, additionally, a CAT reporter was co-transfacted. In case of the luciferase assay a RSV-driven luciferase reporter was co-transfacted (4 µg per reaction) to later standardize the transfection efficiency. The uptake of DNA into cells was increased by a 25% glycerol/10% FBS medium shock and, subsequently, the cells incubated for 48 hours at 37° C until the reporter assays and lyses in the reporter-lyses-buffer (Promega) was performed. Before starting the CAT assay the lysates were checked for their luciferase activity (transfection efficiency) in a luminometer (Fa. Pharmingen) as well as standardized in a BCA assay (Fa. BioRad, Hercules, CA, USA) for the protein concentration.

The luciferase assay was performed following the manual of the Dual Luciferase Reporter Assay Kits (Promega) but using the u-PAR promoter-luciferase reporter. For standardization of the transfection efficiency the cells, which had been co-transfected with a Renilla-Luciferase -Plasmid from Promega were used.

For the analysis of the CAT activity the lysates were incubated for 4 to 6 hours at 37° C with C¹⁴-Chloramphenicol (4 µM, Fa. ICN) and 1 mg/ml acetyl-coencyme A as substrate. The hydrophobic chloramphenicol was then extracted with ethyl acetate and separated in a thin layer chromatography (chloroform/methanol 95:5 as mobile phase). The degree of a 1-times or 2-times acetylated chloramphenicol is representative for the CAT activity and, thus, the promoter activity in the reporter construct. For quantifying of the results a Storm 840 phosphoimager (Molecular Dynamics, Sunnyvale, CA, USA) was used.

It was shown that the PP according to the invention clearly induces a CAT activity in RKO cells, while in GEO cells no CAT activity was detected (Fig. 4 and 5).

### Example 5: Beta-Galactosidase-Vectors

Traditionally the expression of the various promoter polymers in RKO cells was also tested in a beta-galactosidase assay (β-Gal). Therefore beta-Gal vectors with various promoter polymers were generated. The vector beta-GalBasic (Clontech) was digested with BglII, the relevant polymers were digested with PstI and XbaI and all products as well as the vector blunted with T4-DNA-polymerase. To avoid autoligation the Gal-vector were dephosphorylated with calf intestinal phosphatase and, subsequently, for 16 hours at 16° C a ligation of the polymers with the vector in different ratio performed. Products comprising a polymer were identified via an analytic digest using XhoI/HindIII, suitable products were transformed and cloned in mini-preps and, subsequently, sequenced to identify the right orientation.

Furthermore, luciferase vectors were generated. The polymers were tested as described for the Gal-vectors and cloned into pGL3 (Promega) cut with SmaI, blunted and dephosphorylated as described above.

### Example 6: Beta-Galactosidase Assay

The results of the CAT assay as described above were confirmed in another reporter system after recloning the polymers comprising the AP-2/Sp1 region of the u-PAR-promoter in a different reporter system. Also in the beta-galactosidase assay the activation of all 3 polymers in RKO cells, a high expression cell line, was confirmed in comparison to the empty control vector without polymer. In GEO cells (which normally show rare u-PAR expression) no activation of the construct, which would be represented by a detectable beta-Gal value, was shown (Fig. 6).

### Example 7: Cloning strategy for a therapeutic vector

In previously generated vectors in which the CAT gene was regulated by the promoter polymer, this CAT gene was replaced by a therapeutic gene, for example Herpes-TK or an apoptosis gene. In accordance with the CAT assay data it was shown that the expression of the therapeutic gene in the metastatic RKO cells leaded to suicide, but did not in the GEO cells. By using the Herpes TK gene this result was additionally inducibel by the controlled application of gancyclovir.

### Example 8: Cloning strategy of a beta-GAL expressing viral vector

For an *in vivo* testing of the tumor specific expression the polymer that had been introduced in the pCATbasic vector was PCR amplified by using the primers 5'-GGAGGTACCGAGCTCTTACG-3' (sense) (SEQ ID No.: 4) and 5'-GCTCTAGAGCACTAGTGGTGACTTC-3' (reverse) (SEQ ID No.: 5) in a way that 5' of the polymer an additional KpnI restriction site and 3' a new SpeI restriction site a XbaI restriction site were available. Both the cloning vector PUC 19 (Amersham Pharmacia Biotech) as well as the amplified polymer were digested with KpnI and XbaI and ligated for 16 hours at 16° C. Resulting PUC 19 construct comprising the polymer at the previously existing KpnI and XbaI position (PUC-Poly) and additionally, having the newly introduced SpeI restriction side 3' of the polymer. 3' of the newly introduced SpeI site a PstI site from the multiple cloning site of the original plasmid is located. For isolating a suitable LacZ gene for the viral vector the pZn1 vector (Girod et al., Nature Medicine 5(9), pp 1052-56, (1999)) was linearized with BamHI, then blunted and again digested with SpeI in a way that a 3.7 kb fragment (Lac fragment) arises, which had at the 5' end a SpeI site followed by the LacZ gene and 3' thereof a blunted BamHI sequence.

To introduce the LacZ genes 3' of the promoter polymer in the PUC 19 plasmid this PUC 19 plasmid (PUC-Poly) was digested with PstI, blunted and thereafter again digested with SpeI. Into this open SpeI site 3' of the polymer the Lac-fragment can be introduced in sense orientation by ligating the fragment 16 hour at 16° C to the PUC-Poly plasmid. The resulting vector (Poly-Lac-PUC), which now comprises the polymer followed by the LacZ gene and thereafter a multiple cloning site (MCS) of the original PUC-19-plasmid still having the restriction sites SphI and HindIII.

Since it was suggested that the insert of the viral vector should no remain under a certain size, additional non-coding DNA sequence of about 300 base pairs (bp) was introduced into the vector. Therefore, a 330 bp per fragment of the pGL2 plasmid (Promega) was isolated by a BsGI and HpAI digest and subsequently blunted. The vector Poly-Lac-PUC was now linearized with SphI, blunted and the 330 bp non-coding DNA fragment ligated to this side 3' of the LacZ gene. The resulting vector is called: Poly-Lac-PUC-Füll. There is one intact HindIII side 3' of the insert.

To reclone now the polymer together with the LacZ and the non-coding DNA into an AAV-type2-plasmide psub201+ (Samulski et al., 1989). The Psub201+ was digested with XbaI and blunted. Instead of this vector every other viral vector is useful. The previously generated vector Poly-Lac-PUC-Füll was digested with KpnI/HindIII, blunted and religated with the XbaI-digested psub201. Psub201 constructs with a Poly-Lac-Füll insert in sense orientation were identified by sequencing. The generation and packaging of virus was performed as previously published by Samulski et al.

A second strategy to construct virus was to insert the Poly-Lac-Füll-insert into a SnaBI and XhoI-digested psub201⁺-vector via ligation. This vector still comprises the ITR region. The vector is then transfected together with a plasmid expressing the AAV structural genes as well as an adeno-helper-plasmid into 293 cells (described in WO 00/47757). This method guarantees that while generating recombinant AAV viruses no adenoviruses arise.

### Example 9: Generation of a recombinant virus with Herpes Simplex Thymidin Kinase (HSV-TK) Gene

The construction of the therapeutic virus follows these strategy described above whereby the HSV-TK gene is isolated either with an restriction enzyme digest or with a PCR amplification the plasmid comprising the HSV-TK gene for example the plasmid, pAD-CM-TK (Mueller et al., Hamburg). The gene is then blunted and together with the described promoter polymer introduced into the PUC-19 plasmid (Puc-Poly, Promega), which has been digested with PstI and blunted. Constructs having the insert in the right orientation can be identified by sequencing.

The generation of recombinant virus follows the described method.

### References

Aguirre-Ghiso, et al.; J Cell Biol 144: 1285 - 1294, 1999,
Allgayer, H., et al.; Cancer 80: 179 - 187, 1997a.
Allgayer, H., et al.; J Histochem Cytochem 45(2): 203-212, 1997b.
Allgayer, H., et al.; Cancer Research 57: 1394 - 1399, 1997c.
Allgayer, H., et al.; Brit J Surgery 84: 1651-1664, 1997d.
Allgayer, H., et al.; Oncology 55:152 - 160, 1998a.
Allgayer, H., et al.; Clin Exp Metastasis 16:62-73, 1998b.
Allgayer, H et al.; J Biol Chem 274 (8): 4702 - 4714, 1999a.
Allgayer, H., et al.; Journal of Biological Chemistry 274 (26), 18428 - 18437, 1999b.
Allgayer, H, et al.; British Journal of Cancer 80(12), 1884- 1891, 1999c.
Allgayer, H.: Habil thesis, Ludwig-Maximilians-Universität, München, 2001
Andreasen, P. A., et al.; FEBS Lett 338(3): 239 - 245, 1994.
Brattain, M. G et al.; Cancer Metastasis Rev 3: 177 - 191, 1984.
Behrendt, N., et al.; J Biol Chem 265(11): 6453 - 6460, 1990.
Bianchi, E., et al.; Cancer Res 54: 861 - 866, 1994.
Blasi, F.: Fibrinolysis 2: 73 - 84, 1988.
Blasi, F.: BioEssays 15: 105 - 111, 1993.
Bohuslav, J., et al.; J Exp Med 181: 1381 - 1390, 1995.
Borglum, A. D., et al.; Am J Hum Genet 50: 492 - 497, 1992.
Busso, N., et al.; J Cell Biol 126(1): 259 - 270, 1994.
Cantero, D., et al.; Brit J Cancer 75(3): 388 - 395, 1997.
Casey JR, et al.; Blood 84: 1151-1156, 1994.
Cavallaro, U., et al.; J Biol Chem 68(31): 23186 - 23190, 1993.
Conese, M., et al.; J Biol Chem 269(27): 17886 - 17892, 1994.
Cubellis, M. V., et al.; EMBO J 9: 1079 - 1085, 1990.
Dano, K., et al.; Adv Cancer Res 44: 139 - 266, 1985.
Dewerchin, M., et al.; J Clin Invest 97(3): 870 - 878, 1996.
Dignam, J. D, et al.; Nucl Acid Res 11(5): 1475 - 1489, 1983.
Duffy, M. J.; Clin Exp Metastasis 10: 145 - 155, 1992.
Dvorak, H. F.: N Engl J Med 315: 1650 - 1659, 1986.
Ellis, V., et al.; J Biol Chem 266, 12,752-12,758, 1991.
Estreicher, A., et al.; J Cell Biol 111: 783 - 792, 1990.
Gronow, M., et al.; Trends Biotech 1: 26 - 29, 1983.
Günzler, W. A., et al.; J Physiol Chem 363: 133 - 141, 1982a.
Günzler, W. A., et al.; J Physiol Chem 363: 1155 - 1165, 1982b.
Heiss, M. M., et al.; J Clin Oncol 13(8): 2084 - 2093, 1995b.
Hollas, W., et al.; Cancer Res 51: 3690 - 3695, 1991.
Jänicke, F., et al.; Breast Cancer Res and Treatment 24: 195 - 208, 1993.
Jankun, J., et al.; J Cell Biochem 53: 135 - 144, 1993.
Kariko, K., et al.; Cancer Res. 53, 3109-3117, 1993.
Konakova, M., et al.; Eur J Biochem 253: 421 - 429, 1998.
Kook, Y.H., et al.; EMBO J 13, 3983-3991, 1994.
Langer, D. J., et al.; Circulation Research 72: 330 - 340, 1993.
Lengyel, E., et al.; J Biol Chem. 271, 23176-23184, 1996.
Liotta, L. A.: Cancer Res 46: 1-7, 1986.
Liotta, L. A., et al.; Cell 64: 327 - 336, 1991.
Markus, G.: Enzyme 40: 158 - 172, 1988.
May, A. E., et al.; J Exp Med 188(6): 1029 - 1037, 1998.
Min, H. Y., et al.; J Immunol 148: 3636 - 3642, 1992.
Moller, L. B., et al.; Eur J Biochem 208: 493 - 500, 1992.
Morita, S., et al.; Int J Cancer 78: 286 - 292, 1998.
Nekarda, H., et al.; Cancer Res 54(11): 2900 - 2907, 1994.
Nykjaer, A., et al.; J Biol Chem 269(41): 25668 - 25676, 1994a.
Nykjaer, A., et al.; Ann N Y Acad Sci 737: 483 - 485, 1994b.
Nykjaer, A., et al.; J Biol Chem 267: 14543 - 14546, 1992.
Olson, D., et al.; J Biol Chem 267: 9129-9133, 1992.
Osborne, M. P., et al.; Oncology 8: 25 - 31, 1994.
Ossowski, L.: J Cell Biol 107(6): 2427 - 2445, 1988.
Pedersen, T. L., et al.; Br J Haematol 95: 45 - 51, 1996.
Plesner, T., et al. ; Am J Clin Pathol 102(6): 835 - 841, 1994
Ploug, M., et al.; Sem Thromb Hem 17: 183 - 193, 1991a.
Pyke, C., et al.; Cancer Res 51: 4067 - 4071, 1991a.
Pyke, C., et al.; Am J Pathol 138, 1059 - 1067, 1991b.
Pyke, C., et al.; FEBS 326, 69-74, 1993.
Quattrone, A., et al.; Cancer Res 55(1): 90 - 95, 1995.
Reiter, L. S., et al.; Int J Cancer 53: 444 - 450, 1993.
Resnati, M., et al.; EMBO J 15, 1572-1582, 1996.
Riethmüller, et al.; Curr Opin Immunol 4: 647 - 655, 1992.
Riittinen, L., et al.; FEBS Letters 381: 1-6, 1996.
Roldan, A., et al.; EMBO J 9, 467-474, 1990.
Romer, J., et al.; J Invest Dermatol 102(4): 519 - 522, 1994a.
Romer, J., et al.; Int J Cancer 57: 553 - 560, 1994b.
Samulski et al., J. Virol 63, 1989, pages 3822-28.
Schlechte, W., et al.; Cancer Res 49: 6064 - 6069, 1989.
Schlimok, G., et al.; J Clin Oncol 8: 831 - 837, 1990.
Schmitt, M., et al.; Fibrinolysis 6(4. Suppl.): 3 - 26, 1992.
Sier, C. F. M., et al.; Gut 34: 80 - 85, 1993.
Sliutz, G., et al.; Breast Cancer Res Treat 40: 257- 263, 1996.
Soravia, E., et al.; Blood 86: 624-635, 1995.
Stahl, A., et al.; J Cell Biol 129(2): 335 - 344, 1995.
Tang, H, et al.; J Biol Chem 273(29): 18268 - 18272, 1998.
Vagnarelli, P., et al.; Cytogentics and Cell Genetics 60: 197-199, 1992.
Vassalli, J. D., et al.; J Exp Med 159: 1653 - 1668, 1984.
Vassalli, J. D., et al.; J Cell Biol 100: 86 - 92, 1985.
Wang, H., et al.; Int J Cancer 58: 650-657, 1994.
Wang, Y., et al.; Eur J Biochem 227: 116 - 122, 1995.
Wei, Y., et al.; Science 273: 1551 - 1555, 1996.
Wei, Y., et al.; J Biol Chem 269(51): 32380 - 32388, 1994.
Wun, T., et al.; J Biol Chem 257: 7262 - 7268, 1982.
Wun, T. C., et al.; J Biol Chem 257: 3276 - 3283, 1982.
Xue, W., et al.; Cancer Res 57: 1682 - 1689, 1997.
Yebra, M., et al.; Journal of Biology Chemistry 271: 29393-29399, 1996.

## Claims

1. Synthetic promoter polymer comprising two or more repetitions of a regulatory DNA sequence whereby the DNA sequence is a tissue specific and trans-activated regulatory element of the urokinase receptor promoter (u-PAR), and is selected from the group consisting of the sequence areas - 190/-171, -148/-124, -152/-128, -152/-130, -152/-135, -154/- 128, -154/-130 and -154/-135 relative to the transcription initiation site.

2. Synthetic promoter polymer according to claim 1, whereby the regulatory DNA sequence comprises at least one copy of the sequence SEQ ID No.: 1.

3. Synthetic promoter polymer according to any of the claims 1 to 2, whereby the repetitions of the regulatory DNA sequence are separated by a spacer.

4. A vector comprising a marker gene or a therapeutic gene under the regulatory control of the synthetic promoter polymer according to any of the claims 1 to 3.

5. Recombinant virus comprising a marker gene or a therapeutic gene under the regulatory control of the synthetic promoter polymer according to any of the claims 1 to 4.

6. Recombinant virus according to claim 5, whereby the virus is an adeno-associated virus (AAV), an adenovirus or a retrovirus.

7. Pharmaceutical composition comprising the vector according to claim 4 or recombinant virus according to claims 5 or 6 and a pharmaceutical acceptable additive, adjuvant or diluent.

8. The vector according to claim 4 or the recombinant virus according to claims 5 or 6 for the use as medicament.

## Patentansprüche

1. Synthetisches Promoterpolymer enthaltend zwei oder mehr Wiederholungen einer repetitiven DNA-Sequenz, wobei die DNA-Sequenz ein gewebespezifisches und trans-aktiviertes regulatorisches Element des Urukinaserezeptorpromoters (u-PAR) ist, und ausgewählt ist aus der Gruppe bestehend aus den Sequenzarealen -190/-171, -148/-124, -152/-128, -152/-130, -152/-135, -154/-128, -154/-130 und -154/-135 bezogen auf die Transkriptionsinitiationsstelle.

2. Synthetisches Promoterpolymer gemäß Anspruch 1, wobei die regulatorische DNA-Sequenz wenigstens eine Kopie der Sequenz ID Nr.: 1 enthält.

3. Synthetisches Promoterpolymer gemäß einem der Ansprüche 1 oder 2, wobei die Wiederholungen der regulatorischen DNA-Sequenz durch Abstandshaher getrennt sind.

4. Ein Vektor enthaltend ein Marker-Gen oder ein therapeutisches Gen unter der regulatorischen Kontrolle des synthetischen Promoterpolymers gemäß einem der Ansprüche 1 bis 3.

5. Ein rekombinanter Virus enthaltend ein Marker-Gen oder ein therapeutisches Gen unter der regulatorischen Kontrolle des synthetischen Promoterpolymers gemäß einem der Ansprüche 1 bis 4.

6. Rekombinanter Virus gemäß Anspruch 5, wobei das Virus ein Adeno-assoziierter Virus (AAV), ein Adenovirus oder ein Retrovirus ist.

7. Pharmazeutische Zusammensetzung enthaltend den Vektor gemäß Anspruch 4 oder den rekombinanten Virus gemäß Anspruch 5 oder 6 und ein pharmazeutisch annehmbares Zusatzmittel, Adjuvans oder Verdünner.

8. Der Vektor gemäß Anspruch 4 oder der rekombinante Virus gemäß der Ansprüche 5 oder 6 für die Verwendung als Medikament.

## Revendications

1. Un polymère promoteur synthétique comprenant deux ou plus de répétitions d'une séquence ADN régulatrice où la séquence ADN est un élément régulateur transc-activée et spécifique de tissu du promoteur récepteur d'urokinase (u-PAR) et est sélectionné parmi le groupe constitué des aires de séquences -190/-171, -148/-124, -152/-128, -152/-130, - 152/-135, -154/-128, -154/-139, et -154/-135 relativement au site d'initiation de transcription.

2. Un polymère promoteur synthétique selon la revendication 1 où la séquence ADN régulatrice comprend au moins une copie de la séquence ID No. : 1.

3. Un polymère promoteur synthétique selon l'une des revendications 1 à 2 où les répétitions de la séquence ADN de régulation sont séparées par un espaceur.

4. Un vecteur comprenant un gène marqueur ou un gène thérapeutique sous contrôle régulateur du polymère promoteur synthétique selon l'une des revendications 1 à 3.

5. Un virus recombinant comprenant un gêne marker ou un gêne thérapeutique sous contrôle régulateur du polymère promoteur synthétique selon l'une des revendications 1 à 4.

6. Un virus recombinant selon la revendication 5 où le virus est un virus adéno-associé (AAV), un adénovirus ou un rétrovirus.

7. Une composition pharmaceutique comprenant le vecteur selon la revendication 4 ou un virus recombinant selon les revendications 5 ou 6 et un additif, adjuvant ou diluant acceptable pharmaceutique.

8. Le vecteur selon la revendication 4 ou le virus recombinant selon les revendications 5 ou 6 pour l'utilisation comme médicament.
